# EUROPEAN PATENT APPLICATION

(11) **EP 4 534 122 A1**
(43) Date of publication of application: **09.04.2025**
(21) Application number: 23201682.4
(22) Date of filing: 04.10.2023
(51) Int. Cl.: A61M 5/142, A61M 5/14, A61M 39/24

(54) **MEDICAL FLUID DOSING DEVICE**

(71) Applicant: Medizinische Universität Graz, 8010 Graz (AT)
(72) Inventor: REGITTNIG, Werner, 8010 Graz (AT); PIEBER, Thomas, 8010 Graz (AT)
(74) Representative: Gramm, Lins & Partner Patent- und Rechtsanwälte PartGmbB

(57) **Abstract**

This invention relates to a medical fluid dosing device with a cylinder (1, 38) and a piston (2, 39) which is mounted in the cylinder (1, 38) so as to be longitudinally displaceable and forming with the cylinder (1, 38) a working chamber (6) having a variable volume, with an actuator (33, 45) which displaces the piston (2, 39) along the longitudinal extent of the cylinder (1, 38) over the length of a piston stroke, with a fluid reservoir (9) which is fluidically coupled to the cylinder (1, 38) via a supply line (16), with a discharge line (17) which leads from the cylinder (1, 38) to a dispensing point (23), with at least one first valve (13) in the supply line (16), which prevents backflow from the cylinder (1, 38) into the fluid reservoir (9), and at least one second valve (13) in the discharge line (17), which prevents backflow from the dispensing point (23) to the cylinder (1, 38), wherein the cylinder (1, 38) is in the form of a capillary tube and has an internal diameter (d) which is dimensioned such that a vertically oriented cylinder (1, 38) is completely filled with the fluid to be pumped, at least over the length of the piston stroke, counter to the force of gravity by the capillary force alone.

## Description

The invention relates to a medical fluid dosing device with a cylinder and a piston which is mounted in the cylinder so as to be a longitudinally displaceable and forming with the cylinder a working chamber of variable volume, with an actuator which displaces the piston along the longitudinal extent of the cylinder over the length of the piston stroke, with a fluid reservoir, which is fluidically coupled to the cylinder via a supply line, with a discharge line which leads from the cylinder to a dispensing point, with at least one first valve in the supply line which prevents a backflow from the cylinder into the fluid reservoir and at least one second valve in the discharge line which prevents a backflow from the dispensing point to the cylinder. Such a fluid dosing device is designed in particular as a dosing device for medical active substances, in particular insulin.

In case of a metabolic disease such as diabetes, it may be necessary for the patient to administer insulin. For this purpose, patients increasingly use an insulin pump to continuously administer insulin through a fine-bore cannula inserted under the skin. The pumps currently used can be divided into two groups, a tethered pump and a patch pump. Tethered pumps use a thin, flexible tube of various lengths to connect the subcutaneously inserted cannula to the insulin reservoir of the pump. This allows the patient to wear the pump at convenient locations, such as in clothing pockets or on belt clips, and to infuse insulin at more remote locations, such as thighs and upper arms. In contrast to the tethered pump, the patch pump integrates the insulin reservoir, the connecting tubing, the pumping mechanism and the upper part of the subcutaneous cannula into one small device, that can be directly attached to an infusion site on the user's skin. Patch pumps are generally smaller than tethered pumps, but require an additional remote control for setting and programming the amounts of insulin to be delivered.

Contemporary insulin pumps are all designed to administer insulin preparations with a standard concentration of 100 insulin units per millilitre. In recent years, preparations have been developed that have much higher concentrations (for example 500 insulin units per millilitre) and in some cases have similarly fast-acting properties as the conventional insulin preparations. Thus, it would be desirable to administer these new, fast-acting, higher concentration insulin preparations via insulin pumps as well. However, safe use of these new preparations requires a very high degree of accuracy in their administration. Contemporary insulin pumps may not meet these high requirements.

A micropipette is known from US 3,828,987, with which a pre-selected quantity of a fluid can be dispensed. The micropipette comprises a capillary in which a plunger is arranged to aspirate or dispense the fluid.

US 3,951,147 relates to an implantable, refillable pump with an infusion, with an inlet and an outlet and a power cell via which the fluid is forced out of the outlet.

US 4,931,050 relates to an implantable infusion pump that can deliver infusion fluids at different rates and at a constant pressure. A needle is longitudinally displaceable within a capillary. The needle can be displaced over a variable length within the capillary.

US 5,622,482 relates to a pump having a cylinder with an inlet and an outlet and a piston slidably mounted within the cylinder between a first position and a second position to move fluid from the inlet to the outlet. The piston is held in a first position by biasing means. A shape memory alloy element is used as a drive to move the piston from the first position to the second position overcoming the biasing force.

US 8,915,893 B2 relates to implantable devices and in particular implantable pumps of a reduced size which are programmable to deliver a fluid at different rates to a selected site on the human body. A first pressure sensor, a second pressure sensor, an actuating mechanism, a motor, an eccentric cam and a flexible membrane are disposed in a hermetically sealed housing with a sealed interior. A valve unit is in contact with the flexible membrane and is moved by a bending of the membrane due to the movement of the cam.

US 7,959,606 B2 relates to a method for pumping a predetermined dose of insulin with an insulin pump comprising a plunger to perform a reciprocal movement in a cylinder. The cylinder is connected to a supply line and a discharge line, in each of which a check valve is arranged. The plunger is driven via a shape memory alloy.

The object of the present invention is to improve existing fluid dosing devices so that they meet the high requirements for dosing accuracy.

This task is solved by a medical fluid dosing device with the features of the main claim. Advantageous embodiments and further developments of the invention are disclosed in the subclaims, the description and the figures.

The medical fluid dosing device with a cylinder and a piston which is mounted in the cylinder so as to be longitudinally displaceable and forming with the cylinder a working chamber having a variable volume, with an actuator which displaces the piston along the longitudinal extent of the cylinder over the length of the piston stroke, with a fluid reservoir which is fluidically coupled to the cylinder via a supply line, with a discharge line which leads from the cylinder to a dispensing point, with at least one first valve in the supply line which prevents a backflow from the cylinder into the fluid reservoir and at least one second valve in the discharge line which prevents a backflow from the dispensing point to the cylinder, provides that the cylinder is designed as a capillary tube and has an internal diameter which is dimensioned such that, in the case of a vertically oriented cylinder, the cylinder is completely filled with the fluid to be pumped, at least over the length of the piston stroke, against the force of gravity by the capillary force alone. The maximum rise of a fluid in a cylinder due to capillary action is inversely proportional to the cylinder's internal diameter. Thus, in the case of a sufficiently small internal diameter of the cylinder, the fluid to be pumped will move without the assistance of any external forces into the working chamber during a backward movement of the piston. Therefore, because of the capillary action in small diameter cylinders, there is no additional force needed to suck the fluid into the working chamber, and the force to be applied to move the piston backwards is reduced to the force necessary to overcome both the weight of the piston and the friction between the piston and the cylinder. Furthermore, since the fluid can be fully expelled from the working chamber during the forward motion of the piston, the accuracy of the dispensing rate is mainly determined by the manufacturing tolerance in the inner cylinder diameter, i.e. the diameter of the bore in which the piston is moved longitudinally, and the outer piston diameter. Both the cylinder and the piston can, however, be manufactured to sufficiently narrow tolerances. This ensures that permanent, precise metering of even the smallest flow rates is maintained.

In a further development, the internal diameter of the cylinder is dimensioned in such a way that, in the case of a vertically oriented cylinder open on both sides, the cylinder is completely filled with the fluid to be pumped up to a lower reversal point of the piston by capillary force alone. The lower reversal point of the piston is the point at which the working chamber has expanded to its largest possible or previously set desired volume. This ensures that complete filling of the previously set or maximum working chamber volume occurs even if no suction force is exerted by the retracting piston. If the cylinder's internal diameter is dimensioned so that exactly the volume of fluid to be dispensed is moved into the cylinder by the capillary force, the exact required stroke of the piston can be monitored, for example, by a force sensor device that measures whether an additional force is required to suck in the fluid. The lower reversal point of the piston can thus be determined as the distance between the capillary's upper end and the point along the capillary's longitudinal extent at which the sensor has detected a suction force that is exerted by the retracting piston onto the fluid in the capillary.

In one embodiment, the medical fluid dosing device has a connecting element into which the cylinder, the supply line and the discharge line merge. Due to the small dimensions of the cylinder and the supply line as well as the discharge line, the emerging of these device components into a common connecting element is preferable, as the cylinder and its cylinder walls are often too small to be able to form a stable receptacle for the supply line and the discharge line.

In one embodiment, the piston is formed as a wire, wherein a seal is arranged between the cylinder and the wire to seal the working chamber formed by the wire and the cylinder. Advantageously, the seal is attached to the cylinder and slides on the piston during piston movement. The piston is thus a plunger piston which is sealed off from the environment by a seal which is statically arranged in the cylinder or at the cylinder. The result of such an arrangement is that high leakage pressures can be achieved with comparatively low friction. With a seal arranged on the piston and moving relative to the cylinder, a frictional force is generated which acts against the seal, causing a reaction force which counteracts the displacement movement of the piston. The design as a plunger piston takes into account the need to obtain the lowest possible friction in order to achieve an exact displacement with the lowest possible driving forces.

In one embodiment, the internal diameter of the cylinder is between 0.1 mm and 0.9 mm, whereby any diameter between these two limit values can be realised. The respective diameter determines the capillary force generated and thus also the maximum rise of the fluid inside the cylinder, which occurs without suction forces exerted by the piston.

In one embodiment, the clearance or play between the piston and the cylinder is between 1 µm and 10µm, both to ensure free movement of the piston within the cylinder and to minimise leakage or inaccuracy in the metering of the fluid during a piston stroke.

In one embodiment, at least one of the first and second valves is designed as a control valve to effect a controlled, in particular a delayed, release of the fluid from the discharge line or the outlet of the connecting element when a sufficiently high pressure has built up in the working chamber. This prevents the fluid to be dosed from being unintentionally pumped back into the supply line, which would reduce the amount of fluid to be supplied to the patient. In addition, it prevents fluid from being inadvertently administered.

The control valve can be coupled to the actuator of the piston or to a separate actuator. A coupling with the actuator of the piston reduces the size and weight of the fluid dosing device. A separate actuator can be used to achieve independent actuating of the respective control valve.

In one embodiment, the control valves are coupled to a control circuit which opens the first control valve only when the piston has increased the volume of the working chamber and opens the second control valve only when the piston has decreased the volume of the working chamber; i.e., the first control valve in the supply line opens only, after the piston has been moved from an upper end position with a minimum working chamber volume towards a lower reversal position. In particular, the second valve in the discharge line is only opened when the volume of the working chamber has been reduced, i.e. when, after reaching a desired maximum volume of the working chamber, a reversal movement of the piston has already taken place so that an overpressure has set in within the working chamber.

In one embodiment, the fluid dosing device has several cylinders and pistons, whereby the several cylinders and pistons form working chambers of different sizes, which are each connected to the reservoir and the dispensing point. Different sized working chambers are particularly advantageous when a so-called basal delivery and a so-called bolus delivery are to take place. A basal delivery takes place e.g. between meals, for example at night, and comprises a small volume of insulin, while a bolus delivery involves administering a larger amount of insulin immediately before a meal. In order to be able to deliver insulin as quickly as possible during a bolus delivery, the second cylinder has a larger working chamber than the first cylinder, so that the required amount of fluid can be delivered with a small number of piston strokes. The same goes to other fluids to be dispensed in different amounts at specific times.

In one embodiment, the multiple cylinders are attached and fixed to the connecting element and fluidically coupled to the supply line and the discharge line. All cylinders can, for example, merge into a common cavity within the connecting element, so that only one supply line and one discharge line need to be provided.

In a further development, the fluid dosing device has several actuators for several pistons and cylinders, whereby each piston is coupled to an individual actuator. Alternatively, all pistons of the multiple cylinders are coupled to the same actuator, which keeps the installation space of the fluid dosing device small.

In one embodiment, the actuator has a shape memory alloy, an electric motor or a solenoid to drive the piston. A shape memory alloy is comparatively cheap and light and is therefore very suitable for an implanted fluid metering device. In particular, when the piston has to be displaced against a biasing force from an initial position to a working position, a unidirectional shape memory alloy is a favourable drive. The initial position is, for example, the lower piston position in which the working chamber has a maximum volume. A servomotor in the form of an electric motor can easily be used to drive the piston in both directions, so that the piston can be moved to any position by the driving force of the motor. A solenoid valve, relay or solenoid is more complex to construct than a shape memory alloy and has moving parts that can wear out. In the present invention, the inherent imprecise positioning of the piston in a shape memory alloy actuator is compensated for by the defined volume of fluid delivery through the maximum stroke of the piston within the cylinder.

In a further embodiment, independent of the type of drive used, the piston is coupled to a spring which applies a biasing force onto the piston thereby counteracting the backward movement of the piston. When the piston has increased the volume of the working chamber to a maximum value, the spring is released causing a forward movement of the piston and a reduction of the working chamber volume.

In a further development, the piston has two end stops between which it can be moved. The displacement path between the two end stops defines the maximum volume of fluid that can be supplied to the patient with one piston stroke. By positioning the end stops, the respective desired volumes can be set by design. The end stops can be adjustable. An individual adaptation to the respective user or to different models for different fluid volumes can be achieved by adjusting the respective end stop.

All the elements and embodiments described above can be combined with each other.

Examples of embodiments of the invention are explained in more detail below with reference to the figures. The same reference signs indicate the same components and elements. For reasons of clarity, reference signs may have been omitted in some figures. The figures show:
Figure 1 - a schematic representation of a first embodiment as an exploded view;
Figure 2 - a detailed representation of an actuator;

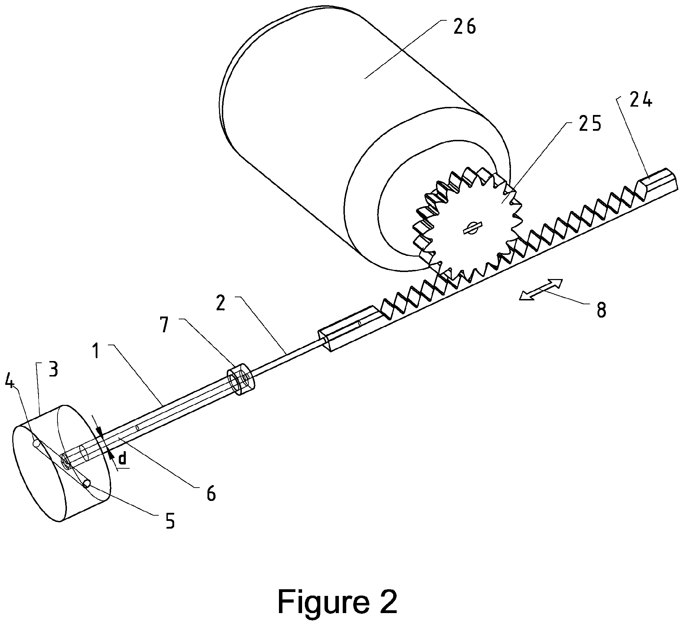

Figure 3A - a variant of Figure 2 with a solenoid of a first position;

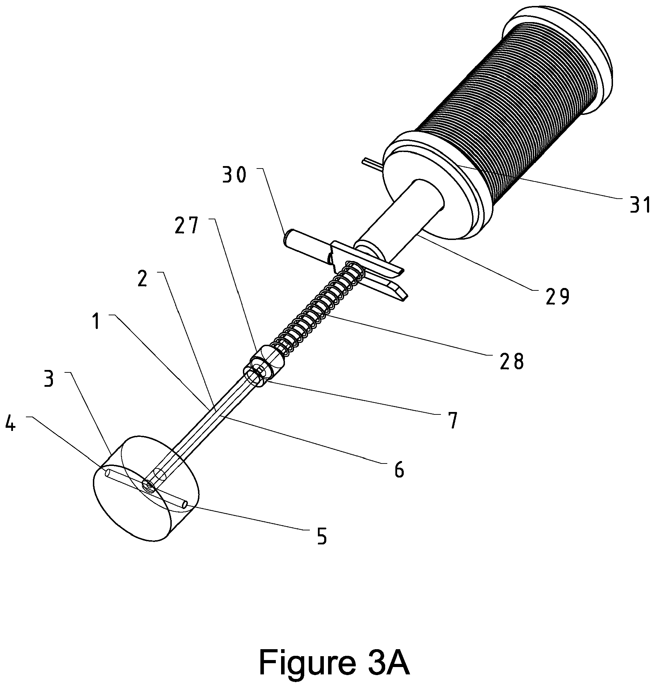

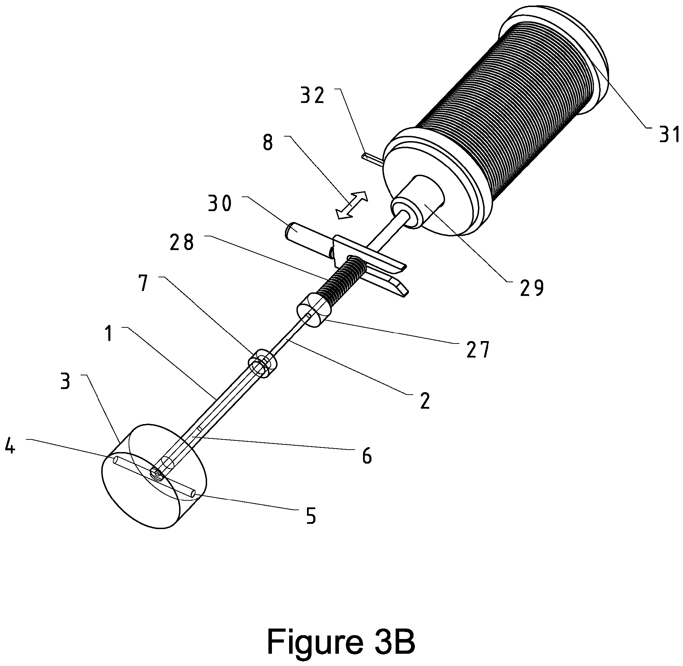

Figure 3B - the assembly according to Figure 3A in a second position;

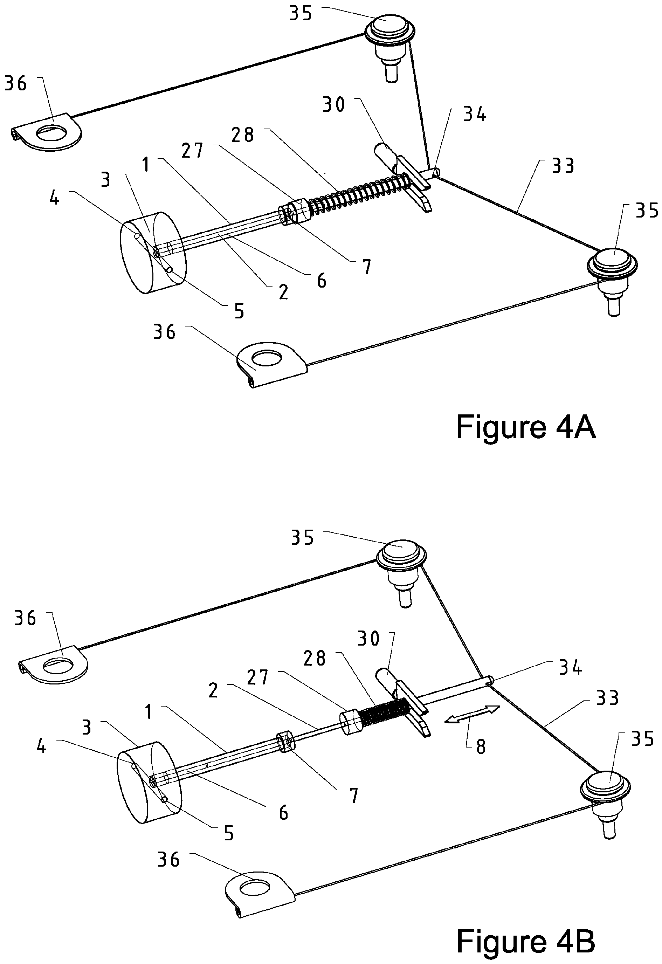

Figure 4A - an embodiment with a shape memory alloy in a first position;
Figure 4B - the structure according to Figure 4A in a second position;
Figure 5A - a variant with two cylinders of different diameters;

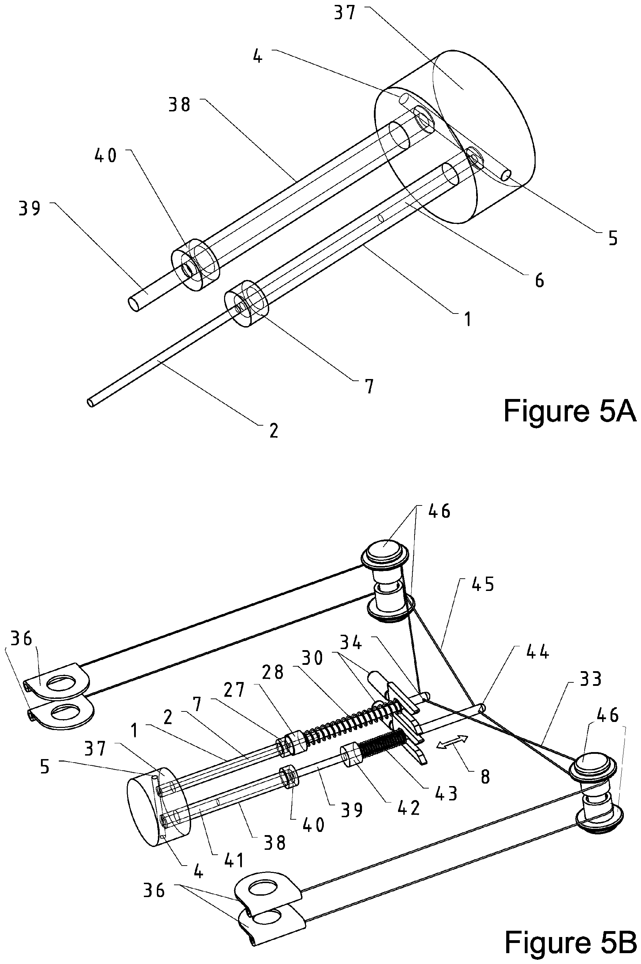

Figure 5 B - the set-up shown in Figure 5A with a shape memory alloy drive;
Figure 6A - a detailed view of a valve in a first position;

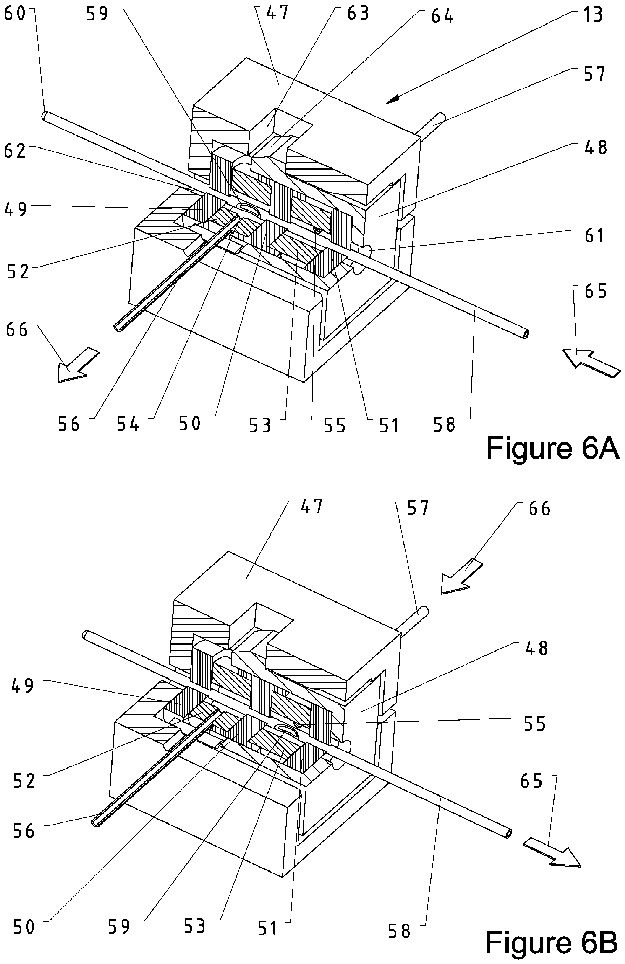

Figure 6B - the valve according to Figure 6A in a second position;
Figure 7A - a control valve with shape memory alloy actuator; and

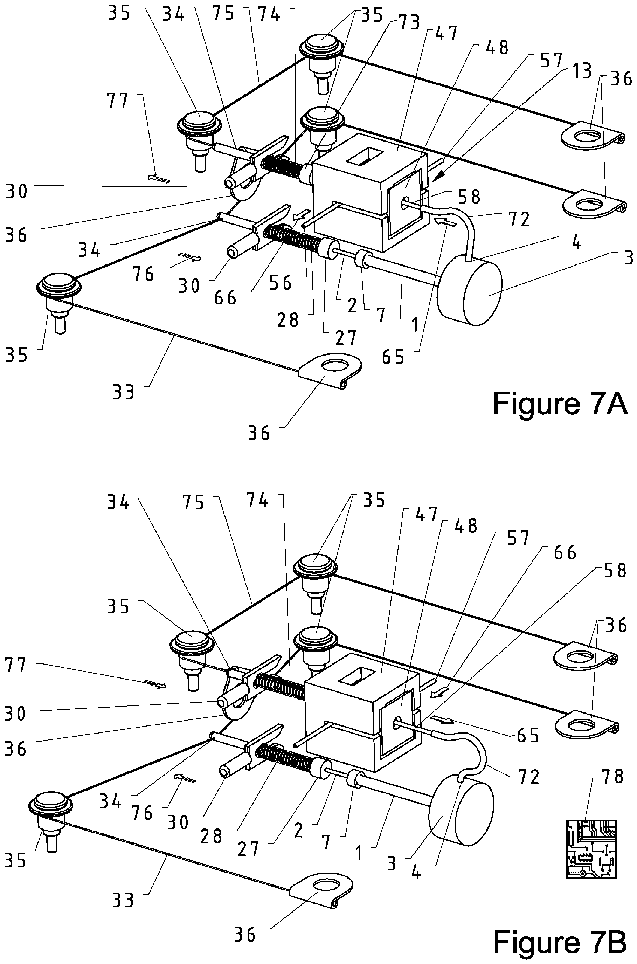

Figure 7B - a control valve according to Figure 7A in a second position.

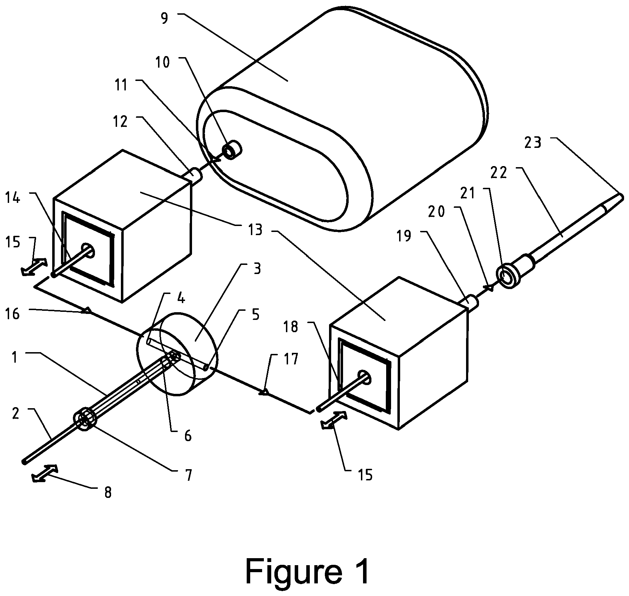

Figure 1 shows a schematic exploded view of a fluid dosing device with a cylinder 1 in which a piston 2 in the shape of a wire as a plunger is arranged to be longitudinally displaceable. The directions of the displacement movement of the piston 2 are shown by the double arrow 8. The cylinder 1 opens into a connecting element 3 which has an inlet 4 and an outlet 5. A supply line 16 opens into the inlet 4, and a discharge line 17 is connected to the outlet 5. The supply line 16 is coupled to a fluid reservoir 9 from which the fluid to be administered, for example insulin or other medication, is directed from a reservoir outlet 10 to an inlet 12 of a control valve 13. The direction of flow is indicated by the arrow 11. The control valve 13 is designed, for example, as a needle valve and directs the fluid to the supply line 16 via a valve outlet. A valve body 14 is displaceably arranged within the valve 13, which is indicated by the double arrow 15. By moving the valve body 14, the valve 13 can be moved from a closed position to an open position. In the open position, fluid can be directed from the fluid reservoir 9 through the supply line 16 to the connecting element 3. When the piston 2 moves away from the connecting element 3, a working chamber 6 formed inside the cylinder 1 enlarges and fluid is conveyed into the enlarging working chamber 6.

A seal 7 is arranged between the cylinder 1 and the piston 2 at the rear end of the cylinder 1 so that the working chamber 6 is sealed off from the environment. The seal 7 is arranged in particular on or in the cylinder 1 and is designed as a static seal in which there is no relative movement of the seal 7 to the cylinder 1. The piston 2 thus operates as a plunger. The fluid dosing device thus represents the embodiment of a plunger pump. In principle, it is also possible to provide the piston 2 with a seal, but this would be technically very complex due to the dimensioning of the components.

If the fluid to be supplied to a patient is in the cavity inside the connecting element 3 between the inlet 4, the outlet 5 and in the working chamber 6, the piston 2 can be displaced in the direction of the connecting element 3 by activating a drive not shown in Figure 1. When the piston 2 is in the position furthest from the connecting element 3, the lower reversal point of the piston 2 is reached. In the opposite position, where the working space 6 is minimal, the piston 2 is in the upper reversal position. If the volume of the working space 6 is reduced by the piston 2, the fluid to be administered is transported from the outlet 5 through the discharge line 17 to a second valve 13 in the discharge line 17. A valve body 18 is also designed to be displaceable in the second valve 13, which is indicated by the double arrow 15. The valve body 18 can be displaced so that the second valve 13 can be switched between a closed position and an open position, intermediate positions are also possible in principle. When the second valve 13 is in an open position and the piston 2 is displaced towards the connecting element 3, the fluid is transported through the second valve 13 to a dispensing point 23, for example a cannula. The dispensing point 23 can be replaceably attached to the second valve 13, for example via a connection piece 21 on an outlet spigot 19, so that the fluid can be transported in the direction of flow 20 through a hollow body 22 to the dispensing point 23.

The cylinder 1 is designed as a capillary tube and has an inner diameter which is dimensioned in such a way that, in the case of a vertically oriented cylinder 1, the capillary is filled with the fluid to be dispensed against the direction of gravity solely by the capillary force. The capillary force acts against gravity and fills the cylinder completely at least over the entire length of a piston stroke. The piston 2 has an outer diameter that is slightly smaller than the inner diameter of the cylinder 1. In particular, the clearance between the cylinder 1 and the piston 2 is between 1 µm and 10 µm, and the piston 2 may be coated on its outer surface. The clearance between the piston 2 and the cylinder 1 is necessary to ensure longitudinal displacement and thus pumping action. The seal 7 may also be arranged on an attachment sleeve at the rear end of the cylinder 1, which is screwed, pressed or otherwise connected to the cylinder 1. The seal 7 and the piston 2 can be supplied together with the attachment sleeve for the seal 7 as a prefabricated, modular component.

Figure 2 shows a detailed view of the actuation of the piston 2 via an electric motor 26. Figure 2 shows that the connecting element 3 has a cylindrical body with a radial transverse bore, the ends of which form the inlet 4 and the outlet 5. An axial bore, in which the cylinder 1 is inserted, merges with the transverse bore, which forms a cavity inside the connecting element 3. The cylinder 1 is formed as a tube and has a cylindrical bore which passes through the cylinder 1 and merges with the transverse bore. Within the bore of the cylinder 1 with an inner diameter d, the piston 2 is mounted for longitudinal displacement and forms the working chamber 6 within the cylinder 1. At the end of the cylinder 1 facing away from the connecting element 3, the seal 7 with the sealing sleeve is arranged.

The end of the piston 2 projecting from the cylinder 1, which is formed as a wire that can also be coated on the outside, is connected to a toothed rack 24 that is coupled to the electric motor 26 via a gear wheel 25. The electric motor 26 can be activated in both directions of rotation so that, depending on the direction of rotation of the electric motor 26, the toothed rack 24 and the piston 2 are displaced in longitudinal extension in one direction or the opposite direction. This is indicated by the double arrow 8. The maximum displacement length of the piston 2 is defined by the length of the toothed rack 24 and/or is limited by a corresponding control connected to the electric motor 26. If the piston 2 is moved out of the cylinder 1 to the maximum extent, the working chamber 6 is enlarged and the fluid to be supplied to the patient is drawn from the fluid reservoir shown in Figure 1. Due to the design of the cylinder 1 as a capillary with an internal diameter d, through which the fluid is transported against the force of gravity into the maximally enlarged working chamber 6 even without suction forces exerted by the piston 2, a low drive power is required to only overcome the friction between the piston 2 and the cylinder 1 as well as the weight of the piston 2 and the toothed rack 24. If the fluid in the working chamber 6 is to be supplied to the patient, the electric motor 26 is reversed and the piston 2 is moved into the cylinder 1 in the direction of the upper end position. The full piston stroke defines the volume of fluid to be supplied. Depending on the setting and control, the necessary or desired quantity is supplied to the patient by activating the drive 26 once or several times in both directions. If the required quantity cannot be achieved by complete piston strokes, the electric motor 26 can also, for example, only provide a partial piston stroke in which the piston 2 is moved in increments less than the distance between the upper and lower reversal point of the piston 2.

Figure 3A shows basically the same construction as in Figure 2, but a solenoid 31 is used as the drive, which has a plunger 29 that is connected to the piston 2. A bias spring 28 is arranged between a spring seat 27 and a spring end plate 30 and drives the piston 2 into a preloaded position. The initial position can be either the lower reversal point or the upper reversal point. The spring end plate 30 retains and preloads the spring 28. The spring seat 27 is attached to the piston 2 and allows appropriate biasing or assists movement by the spring 28. In Figure 3A, the piston 2 is in the maximum extended position, i.e. the upper reversal position, where the working space 6 is minimal.

Figure 3B shows the assembly as in Figure 3A in the maximum retracted position of the piston 2. The spring seat 27 has moved away from the cylinder 1, as can be seen from the distance between the spring seat 27 and the seal 7. The spring 28 is compressed between the spring seat 27 and the spring end plate 30. When a movement of the piston 2 is caused towards the connecting element 3, the spring 28 relaxes and supports the pumping movement of the piston 2.

Figure 4A shows the design with the spring-loaded piston 2 between a spring seat 27 and a spring end plate 30 and the spring 28 arranged between them as shown in Figure 3A. Instead of a solenoid as the drive, the piston 2 is displaced by a wire system with pulleys 35. A wire 33 made of a shape memory alloy is fixed by two wire clamps or wire crimps 36 and tensioned in a U-shape by the pulleys 35. The shape memory alloy wire 33 is passed through a through hole 34 or another receptacle within a sleeve or rod to which the piston 2 is connected. In principle, a one-piece design of the piston 2 with the receptacle 34 for the shape memory alloy wire 33 is also possible. In the illustration of Figure 4A, the wire 33 of shape memory alloy material is cold or below its transformation temperature and therefore in the extended state, so that the spring 28 is relaxed and has moved the piston 2 to the upper end position. When the wire 33 is heated, for example by an electric current, the material of the wire 33 is transformed into a contracted state through which the spring 28 is tensioned, as shown in Figure 4B.

A variant of the fluid dosing device design is shown in Figure 5A, in which the connecting element 37 is connected to two cylinders 1, 38. The transverse bore between the inlet 4 and the outlet 5 is in fluidic connection with two working spaces formed by the pistons 2, 39. Static seals 7, 40 are arranged on both cylinders 1, 38. In the embodiment example shown, the left cylinder 38 is provided with a larger inner diameter than the right cylinder 1. Accordingly, the outer diameter of the left-hand piston 39 is larger than that of the right-hand piston 2. In Figure 5A, the left-hand piston 39 is at the upper reversal point, while the right-hand piston 2 is at the lower reversal point with a maximally enlarged working chamber 6. By moving the pistons 2, 39 accordingly, different large amounts of fluid are moved from the working spaces 6, 41 from the inlet 6 to the outlet 5 and thus to the dispensing point 23, which is not shown. The pistons 2, 39 can be moved individually or together. Independent movement is also possible. The pistons 2, 39 can each be actuated by a separate actuator, or alternatively they can be actuated by a single, common actuator with a changeover device which optionally connects the actuator to one or other of the pistons 2, 39.

Figure 5B shows the configuration of Figure 5A with two separate actuators made of a shape memory material as shown in Figure 4A. The first piston 2 is in the upper reversed position with a minimum working chamber with a cold wire 33, while the second piston 39 in the larger cylinder 38 is moved via a separate wire 45, which is contracted by heating, into the retracted position with a preloaded spring 43 between the spring end plate 30 and the second spring seat 42. The movement of the pistons 2, 39 is shown by the double arrow 8. Two pairs of pulley wheels 46 are provided to deflect the respective wire 33, 45 so that independent displacement of the pistons 2, 39 can take place.

Figure 6A shows a sectional view of the schematic structure of a control valve 13 as shown in Figure 1 with a valve body 47 having a first septum 51, a second septum 50 and a third septum 49 in a septum retainer 48. A first spacer sleeve 52 is sandwiched between the first septum 51 and the second septum 50 and has a side port 54. A second spacer sleeve 53 is sandwiched between the second septum 50 and the third septum 49 and has a side port 55. The side port 54 of the first spacer sleeve 52 is coupled to an outlet component 56, and the side port 55 of the second spacer sleeve 53 is coupled to an inlet component 57. The outlet component 56 may comprise a rigid tube component insertable into the side port 54 of the first spacer sleeve 52. The inlet component 57 may also comprise a rigid tube component insertable into the side port 55 of the second spacer sleeve 53. A valve cannula 58 has a side port 59 and a closed end 60, the closed end 60 may be created by crimping, welding, otherwise sealing and/or filling. An opening 61 in the septum retainer 48 allows entry of the valve cannula 58 such that the open end of the valve cannula 58 protrudes from the valve housing 47. Fluid flows into the open end of the valve cannula 58, as shown by the arrow 65, and exits laterally out of the outlet component 56 from the valve housing 47, as shown by the arrow 66. The housing 47 includes an aperture 63 at the top, into which a projection on the top of the septum retainer 48 can engage to positively lock the septum retainer 48 in the housing 47. On the side of the septum retainer 48 opposite the opening 61, a further opening 62 is formed through which the closed end 60 of the valve cannula 58 can pass. In the position shown in Figure 6A, fluid can be delivered from either the reservoir 9 or the working chamber 6 to the valve 13 and passes through the outlet component 56 from the valve body 47 to the respective desired location, for example the supply line 17 to the delivery point 23 or to the cylinder 1.

In the position shown in Figure 6B, in which the valve cannula 58 is displaced so that the side port 59 of the cannula 58 is located in the region of the second spacer sleeve 53, fluid enters the side port 55 of the second spacer sleeve 53 through the inlet component 57 and allows flow out of the open end of the cannula 58. One possible connection is that the supply line 16 from the reservoir 9 is connected to the inlet component 57, while the discharge line 17 is connected to the outlet component 56.

Figures 7A and 7B show the medical fluid dosing device with a 2-way valve with a shape memory alloy as the actuator in two positions. In Figure 7A the fluid is expelled, in Figure 7B the valve 13 is in the position in which the fluid is sucked in. The structure of the fluid dosing device in Figures 7A and 7B provides for a cylinder 1 as a capillary with a plunger wire 2 as a piston, whereby the cylinder 1 with the working chamber opens into the connecting element 3. A fluid channel 4 opens into the connecting element 3 and connects the connecting element 3 to the control valve 13 as shown in Figures 6A and 6B. The plunger wire 2 is sealed off from the cylinder 1 by the seal 7 and is supported by the bias spring 28, which is elastically prestressed between the valve seat 27 arranged on the plunger wire 2 and the spring end plate 30. To displace the plunger wire 2, the wire 33 from a shape memory alloy element is coupled in a through hole 34 or other receptacle within a sleeve or rod connected to the plunger wire 2.

The basic construction and operation of the pump is the same as described in connection with Figures 4A, 4B as well as 5B. Heating or cooling the wire 33 causes the plunger wire 2 to move in one direction or the other as indicated by the arrow 76. In Figure 7A, both the spring seat 27 and the plunger wire 2 move towards the connector 3. In Figure 7B, assisted by the biasing spring 28, the plunger wire 2 is moved away from the connector 3 together with the valve seat 27 by heating the wire 33. This pulls the plunger wire 2 out of the cylinder 1 and the bias spring 28 is tensioned. When the wire 33 cools and relaxes, the spring 28 also relaxes and fluid is pumped out of the working chamber into the connecting element 3 into a fluid channel 4. The fluid channel 4 acts in this situation as an outlet and directs the fluid from the connector 3 through a flexible pipe connection 72 to the open end of the valve cannula 58 as shown in Figure 6A and indicated by the arrow 65. Fluid exits the control valve 13 from the outlet component 56. The outlet component 56 may be a rigid tube positioned within the side port 54 of the first spacer sleeve 52. The valve cannula 58 is coupled with its closed end 60 to a wire 75, which is also made of a shape memory alloy and acts as a second, independent drive. One end of the wire 75 is positioned at the receptacle 34 at the rear closed end 60 of the valve cannula 58. A biasing spring 74 is disposed between a second spring end plate 30 and a second spring seat 73 and drives the valve cannula 58 towards the position shown in Figure 7B. Both wires 33, 75 are deflected by pulleys 35 and are fixed at their free ends by wire clamps 36. When the second wire 75 is heated, as shown in Figure 7A, the wire 75 moves the valve cannula 58 towards the second spring end plate 30 as shown by arrow 77, so that when the plunger wire 2 moves accordingly, fluid is pumped through the open end of the valve cannula 58 into the control valve 13 and discharged from the outlet component 56.

If the fluid is to be drawn from the reservoir, which is not shown, the first wire 33 is heated so that the plunger wire 2 is pulled out of the cylinder 1. At the same time, or with a time delay via a corresponding control 78, the second wire 75 is cooled so that the valve cannula 58 is displaced according to arrow 77 and the second valve seat 73 is displaced towards the valve housing 47. Accordingly, the fluidic connection to the outlet component 56 is closed and the fluidic connection to the inlet component 57 is opened, so that fluid from the reservoir can flow into the control valve 13 according to the direction of arrow 66 in Figure 7B and be sucked into the working chamber inside the cylinder 1. Depending on the position of the valve cannula 58, fluid is transported from the reservoir to the inlet component 57 into the septum retainer 48 and from there through the open end through the flexible line 72 into the connection element 3 and into the working chamber or from the working chamber through the flexible line 72 and the valve cannula 58 into the septum retainer 48 and from there through the outlet component 56 to the discharge point.

## Claims

1. Medical fluid dosing device with a cylinder (1, 38) and a piston (2, 39) which is mounted in the cylinder (1, 38) so as to be longitudinally displaceable and forming with the cylinder (1, 38) a working chamber (6) having a variable volume, with an actuator (33, 45) which displaces the piston (2, 39) along the longitudinal extent of the cylinder (1, 38) over the length of a piston stroke, with a fluid reservoir (9) which is fluidically coupled to the cylinder (1, 38) via a supply line (16), with a discharge line (17) which leads from the cylinder (1, 38) to a dispensing point (23), with at least one first valve (13) in the supply line (16), which prevents backflow from the cylinder (1, 38) into the fluid reservoir (9), and at least one second valve (13) in the discharge line (17), which prevents backflow from the dispensing point (23) to the cylinder (1, 38), **characterised in that** the cylinder (1, 38) is in the form of a capillary tube and has an internal diameter (d) which is dimensioned such that a vertically oriented cylinder (1, 38) is completely filled with the fluid to be pumped, at least over the length of the piston stroke, counter to the force of gravity by the capillary force alone.

2. Medical fluid dosing device according to claim 1, **characterised in that** the internal diameter (d) is dimensioned in such a way that, in the case of a vertically oriented cylinder (1, 38) open on both sides, the cylinder (1, 38) is completely filled counter to the force of gravity with the fluid to be pumped solely by capillary force up to a lower reversal point of the piston (2, 39).

3. Medical fluid dosing device according to claim 1, **characterised in that** the cylinder (1, 38), the supply line (16) and the discharge line (17) merge into a connecting element (3, 37).

4. Medical fluid dosing device according to claim 1, **characterised in that** the piston (2, 39) is formed as a wire and a seal (7, 40) is arranged around the piston (2, 39) and seals the working chamber (6).

5. Medical fluid dosing device according to claim 4, **characterised in that** the seal (7, 40) is attached to the cylinder (1, 38) and the piston (2, 39) slides along the seal (7, 40) during piston movement.

6. Medical fluid dosing device according to claim 1, **characterised in that** the internal diameter (d) of the cylinder (1, 38) is between 0.1 mm and 0.9 mm.

7. Medical fluid dosing device according to claim 1, **characterised in that** there is a clearance of between 1µm and 10µm between the piston (2, 39) and the cylinder (1, 38).

8. Medical fluid dosing device according to claim 1, **characterised in that** at least one of the first and second valves (13) is designed as a control valve.

9. Medical fluid dosing device according to claim 8, **characterised in that** the control valve (13) is coupled to the actuator (33, 45) of the piston (2, 39) or to a separate actuator (75).

10. Medical fluid dosing device according to claim 9, **characterised in that** the control valves (13) are coupled to a control circuit (78) which opens the first control valve (13) only when the piston (2, 39) has increased the working chamber (6) and opens the second control valve (13) only when the piston (2, 39) has decreased the working chamber (6).

11. Medical fluid dosing device according to claim 1, **characterised in that** several cylinders (1, 38) and pistons (2, 39) with differently sized working chambers (6, 41) are connected to the reservoir (9) and the dispensing point (23).

12. Medical fluid dosing device according to claim 11, **characterized in that** said plurality of cylinders (1, 38) are fixed to said connecting member (3, 37) and fluidically coupled to said supply line (16) and said discharge line (17).

13. Medical fluid dosing device according to claim 11, **characterised in that** each piston (2, 39) is coupled to its own actuator (26, 31, 33, 45) or all pistons (2, 39) are coupled to the same actuator (26, 31, 33).

14. A medical fluid metering device according to claim 1, **characterised in that** the actuator comprises a shape memory alloy (33, 45), an electric motor (26) or a solenoid (31).

15. A medical fluid metering device according to claim 1, **characterised in that** the piston (2, 39) is coupled to a spring (28, 43) which exerts a force that biases the piston (2, 39) counter to a retraction direction of the piston (2, 39).

16. Medical fluid dosing device according to claim 1, **characterized in that** the piston (2, 39) has two end stops (27, 42, 30) between which it is moved.
